# EUROPÄISCHE PATENTSCHRIFT

(11) **EP 2 054 104 B1**
(45) Veröffentlichungstag und Bekanntmachung des Hinweises auf die Patenterteilung: **05.10.2016**
(21) Anmeldenummer: 07801801.7
(22) Anmeldetag: 22.08.2007
(51) Int. Cl.: A61M 1/14, A61M 1/34, A61M 1/16, A61M 1/36, H04L 9/32

(54) **EXTRAKORPORALE BLUTBEHANDLUNGSVORRICHTUNG UND VERFAHREN ZUR VORBEREITUNG EINER BLUTBEHANDLUNG MIT EINER EXTRAKORPORALEN BLUTBEHANDLUNGSVORRICHTUNG**
EXTRACORPOREAL BLOOD TREATMENT DEVICE AND METHOD FOR PREPARING BLOOD TREATMENT USING AN EXTRACORPOREAL BLOOD TREATMENT DEVICE
DISPOSITIF DE TRAITEMENT SANGUIN EXTRACORPOREL ET PROCÉDÉ DE PRÉPARATION D'UN TRAITEMENT SANGUIN AU MOYEN D'UN DISPOSITIF DE TRAITEMENT SANGUIN

(30) Priorität: 26.08.2006 DE 102006040179
(43) Veröffentlichungstag der Anmeldung: 06.05.2009
(73) Patentinhaber: Fresenius Medical Care Deutschland GmbH, 61352 Bad Homburg v.d.H. (DE)
(72) Erfinder: BALSCHAT, Klaus, 97525 Schwebheim (DE); KOPPERSCHMIDT, Pascal, 97456 Dittelbrunn (DE); SPICKERMANN, Reiner, 97535 Wasserlosen-Burghausen (DE)
(74) Vertreter: Oppermann, Frank
(86) Internationale Anmeldenummer: PCT/EP2007/007369
(87) Internationale Veröffentlichungsnummer: WO 2008/025467

(56) Entgegenhaltungen:
- WO-A-01/45767
- WO-A-90/14850
- US-A1- 2006 184 084

## Beschreibung

Die Erfindung bezieht sich auf eine extrakorporale Blutbehandlungsvorrichtung, die über eine interne Kommunikationseinheit zur Kommunikation mit einer externen Kommunikationseinheit verfügt. Darüber hinaus betrifft die Erfindung eine Anordnung aus einer extrakorporalen Blutbehandlungsvorrichtung, die über eine interne Kommunikationseinheit verfügt, einem Kominunikationsnetz und einer externen Kommunikationseinheit zur Kommunikation mit der internen Kommunikationseinheit über das Kommunikationsnetz. Des weiteren betrifft die Erfindung ein Verfahren zur Vorbereitung einer Blutbehandlung mit einer extrakorporalen Blutbehandlungsvorrichtung, die eine interne Kommunikationseinheit zur Kommunikation mit einer externen Kommunikationseinheit aufweist.

Als Verfahren zur extrakorporalen Blutbehandlung sind die Hämodialyse, Hämofiltration und Hämodiafiltration bekannt. Die Abhängigkeit von den extrakorporalen Blutbehandlungsvorrichtungen, beispielsweise den Dialysemaschinen, schränkt die Lebensqualität des Patienten ein.

Die Hämodialyse kann in Dialysezentren oder auch zu Hause (Heim-Dialyse) durchgeführt werden. Während in den Dialysezentren die Dialysemaschine von Klinikpersonal betrieben wird, hat der Patient bei der Heim-Dialyse die Dialysemaschine eigenständig zu betreiben. Die Heim-Dialyse hat aber den Vorteil, dass die Dialyse in der gewohnten Umgebung des Patienten durchgeführt werden kann, wodurch die Lebensqualität des Patienten verbessert wird.

Aus der US 7,044,927 ist ein extrakorporales Blutbehandlungssystem bekannt, dass über eine extrakorporale Blutbehandlungsvorrichtung verfügt, die eine Kommunikation über ein öffentliches Kommunikationsnetz vorsieht, so dass beispielsweise mehrere Dialysemaschinen von einer Zentrale aus überwacht werden können. Darüber hinaus besteht die Möglichkeit, ein Service-Center einzurichten, in dem ein ausgewählter Service-Techniker für Wartungsarbeiten die Komponenten der Maschine überwachen und Einstellungen an der Maschine vornehmen kann. Ferner ist es möglich, Daten zu übertragen, beispielsweise patientenbezogene Informationen, um an einem anderen Ort über den Zustand des Patienten informiert zu sein. Das bekannte Blutbehandlungssystem macht von einem Web-Server und Web-Browser Gebrauch. Der Zugang zu der Blutbehandlungsvorrichtung erfordert eine entsprechende Autorisierung.

Aus der US 2004/0203961 ist bekannt, dass Heim-Dialyse-Patienten im Rahmen der Patientenüberwachung mittels eines Mobil-Telefons eine Kurznachricht (SMS) an das Betreuungspersonal senden. Das Senden eines SMS soll aber nur der Information des Betreuungspersonals dienen. An die Dialysemaschine selbst werden keine Daten übermittelt.

Die EP 1 101 437 A1 und EP 1 226 781 A2 beschreiben ein medizinisches System zur Überwachung von Parametern eines Patienten in häuslicher Umgebung, bei dem der Arzt in dringenden Fällen per E-Mail benachrichtigt wird oder Nachrichten per SMS zur Informationszwecken übermittelt werden.

Außerhalb des Bereiches der Medizintechnik ist beispielsweise bekannt, über ein öffentliches Kommunikationsnetz zum Zwecke der Zeiterfassung für Service- und Abrechnungszwecke Daten zwischen einem Provider und der Maschine zu übermitteln.

Der Betrieb der Dialysemaschine bedarf bestimmter zeitintensiver Prozesse zur Vorbereitung der Behandlung. Zu Beginn der Behandlung ist es beispielsweise erforderlich, die Versorgung der Dialysemaschine mit den verschiedenen Flüssigkeiten sicherzustellen. Hierzu gehören insbesondere die Spülung und Befüllung der einzelnen Komponenten mit Spülflüssigkeit und Dialysierflüssigkeit. Darüber hinaus werden verschiedene Testroutinen durchgeführt, um während der Blutbehandlung einen sicheren Betrieb der Dialysemaschine zu gewährleisten. Bei den Dialysemaschinen wird im Allgemeinen angestrebt, die zur Vorbereitung der Dialysebehandlung erforderlichen Prozeduren möglichst vollständig zu automatisieren.

Während der Vorbereitung der Dialysemaschine ist die Anwesenheit des Patienten nicht unbedingt erforderlich. Da die Vorbereitung der Maschine zeitintensiv ist, bedeutet die Anwesenheit des Patienten einen weiteren Verlust an Lebensqualität. Daher wird angestrebt, die Vorbereitung der Dialyse in Abwesenheit des Patienten automatisch durchzuführen. Damit stellt sich aber das Problem, dass der Patient genau seinen Tagesablauf planen muss, um pünktlich nach Beendigung der Vorbereitungsprozeduren anwesend zu sein. Denn ein verspäteter Behandlungsbeginn hätte eine erhebliche Verschwendung an Betriebsmitteln zur Folge.

Insbesondere die Lebensqualität von Heim-Dialyse-Patienten ließe sich weiter verbessern, wenn sich der Dialyse-Patient während der Vorbereitung der Dialysemaschine nicht zu Hause aufhalten bräuchte.

Die US 2006/0184084 A1 beschreibt eine Anordnung, die eine Dialysevorrichtung und mehrere externe Eingabe-/Ausgabegeräte umfasst. Die Ein- und Ausgabegeräte kommunizieren mit der Dialysevorrichtung über drahtlose Verbindungen. Das Netzwerk soll unter anderem der Vereinfachung der Initialisierung einer Dialysebehandlung dienen.

Eine Vernetzung von extrakorporalen Blutbehandlungseinrichtungen und Ein- und Ausgabegeräten ist auch aus der JP 11033110 A bekannt.

Die EP 0 428 676 B1 beschreibt eine Dialysevorrichtung, die über eine Einrichtung zur Verarbeitung und Speicherung von einzelnen Dialyseparametern zugeordneten Signalen verfügt. Die bekannte Dialysevorrichtung weist eine Zeitschaltuhr auf, mit der die zeitliche Abfolge der Auswahl der einzelnen Werte durch die Vorrichtung zur Verarbeitung und Speicherung eingestellt werden kann. Beispielsweise kann ein Zeitpunkt festgelegt werden, zu dem die Behandlung beginnt.

Die WO 01/45767 A beschreibt eine extrakorporale Blutbehandlungsvorrichtung, die über eine Fernbedienung verfügt. Bei der Fernbedienung handelt es sich um einen als Handgriff ausgebildeten Schalter mit einem Druckknopf, der mit dem Daumen betätigt wird. Die Fernbedienung ist über ein Kabel mit der Steuereinrichtung der Blutbehandlungsvorrichtung fest verbunden. Durch Drücken des Schalters können verschiedene Komponenten der Blutbehandlungsvorrichtung gestartet und/oder gestoppt werden. Beispielsweise kann die Blutpumpe gestartet werden. Durch Starten der Blutpumpe mit der Fernbedienung soll auch ein Spülvorgang eingeleitet werden können, wobei die Pumpe dann anstelle von Blut Spülflüssigkeit fördert.

Der Erfindung liegt daher die Aufgabe zu Grunde, eine extrakorporale Blutbehandlungsvorrichtung bereitzustellen, die den Patienten einen möglichst großen Freiraum bei der Planung des Tagesablaufs erlaubt. Eine weitere Aufgabe der Erfindung liegt darin, ein Blutbehandlungssystem und ein Verfahren zur Vorbereitung einer Blutbehandlung mit einer extrakorporalen Blutbehandlungsvorrichtung anzugeben, das dem Patienten eine möglichst freie Planung seines Tagesablaufs ermöglicht.

Die Lösung dieser Aufgabe erfolgt erfindungsgemäß mit den Merkmalen der Patentansprüche 1, 6 und 8. Vorteilhafte Ausführungsformen der Erfindung sind Gegenstand der Unteransprüche.

Die erfindungsgemäße extrakorporale Blutbehandlungsvorrichtung verfügt wie jede konventionelle Blutbehandlungsvorrichtung über eine Behandlungseinheit zur Durchführung der extrakorporalen Blutbehandlung. Neben der eigentlichen Behandlungseinheit weist die erfindungsgemäße Blutbehandlungsvorrichtung eine Einrichtung zur Steuerung der Behandlungseinheit für die Durchführung der extrakorporalen Blutbehandlung und eine Einrichtung zur Steuerung der Behandlungseinheit für die Vorbereitung der extrakorporalen Blutbehandlung auf. Mit der letzteren Einrichtung werden automatisierbare Prozesse durchgeführt, die zur Vorbereitung der Blutbehandlung erforderlich sind. Hierzu gehören beispielsweise Spül-, Befüllungs- und Testroutinen.

Darüber hinaus verfügt die erfindungsgemäße Blutbehandlungsvorrichtung über eine interne Kommunikationseinheit zur Kommunikation mit einer externen Kommunikationseinheit über ein Kommunikationsnetz. Die interne Kommunikationseinheit wirkt derart mit der Einrichtung zur Steuerung der Behandlungseinheit für die Vorbereitung der extrakorporalen Blutbehandlung zusammen, dass nach dem Empfang eines Initiierungscodes von der externen Kommunikationseinheit die Einrichtung zur Vorbereitung der Blutbehandlung gestartet wird.

Der Patient kann bei der erfindungsgemäßen Blutbehandlungsvorrichtung durch Senden eines Initiierungscodes mit der externen Kommunikationseinheit den Zeitpunkt bestimmen, wann die Blutbehandlungsvorrichtung für die Behandlung vorbereitet wird. Es wird angenommen, dass die Routinen zur Vorbereitung der Behandlung beispielsweise einen Zeitraum von 30 Minuten benötigen. In diesem Zeitraum braucht der Patient nicht anwesend zu sein, so dass er den Tagesablauf frei bestimmen kann. Erst wenn der Patient absehen kann, dass er innerhalb von 30 Minuten für die Heim-Dialyse zu Hause sein kann, startet der Patient die Vorbereitung der extrakorporalen Blutbehandlung, indem er einen Initiierungscode sendet, so dass die Behandlungseinheit für die Vorbereitung der Blutbehandlung in Gang gesetzt wird.

Bei der internen und externen Kommunikationseinheit kann es sich um bekannte Kommunikationseinheiten handeln, die eine Übermittlung des Initiierungscodes über eine Leitung oder eine Funkstrecke eines Kommünikationsnetzes erlauben.

Bei einer bevorzugten Ausführungsform der Erfindung ist die interne Kommunikationseinheit als Mobil-Telefon-Einheit und die externe Kommunikationseinheit als Mobil-Telefon ausgebildet, wobei der Initiierungscode eine Kurznachricht im SMS-Format ist. Durch das Senden einer bestimmten SMS kann der Patient also jederzeit Vorbereitungsroutinen starten.

Grundsätzlich ist es möglich, sämtliche Routinen zur Vorbereitung der Blutbehandlung mit einer SMS zu starten. Es ist aber auch möglich, nur einzelne Vorbereitungsroutinen zu starten. Beispielsweise können verschiedene SMS vorgesehen sein, um zwischen unterschiedlichen Initiierungsroutinen zu unterscheiden.

Eine weitere bevorzugte Ausführungsform sieht vor, dass der Initiierungscode einen Code zur Identifikation eines bestimmten Patienten enthält. Die interne Kommunikationseinheit der Blutbehandlungsvorrichtung verfügt über eine Einrichtung zum Speichern eines Codes zur Identifikation eines bestimmten Patienten und eine Vergleichseinheit zum Vergleichen des in dem Initiierungscode enthaltenden Codes zur Identifikation des Patienten mit dem gespeicherten Identifikationscode. Wenn der gespeicherte Code mit dem Code, der in dem Initiierungscode enthalten ist, übereinstimmt, wird der Patient als identifiziert angesehen. Dadurch kann ein Missbrauch ausgeschlossen werden.

Für den Fall, dass die interne Kommunikationseinheit als Mobil-Telefon-Einheit und die externe Kommunikationseinheit als Mobil-Telefon ausgebildet sind, erübrigt sich grundsätzlich die Identifikation eines bestimmten Patienten insofern, als bei einem Anruf mit einem Mobil-Telefon die Mobil-Telefon-Einheit im Allgemeinen als solche bereits durch die Telefonnummer identifiziert wird. Der Identifikationscode kann aber auch eine nur dem Patienten bekannte zusätzliche alphanumerische Zeichenfolge sein, die Teil der SMS ist.

Wenn das externe Mobil-Telefon ein konventionelles Mobiltelefon ist, kann die interne Mobil-Telefon-Einheit eine modulare Einheit sein, die nur das Empfangen, nicht aber Senden von Kurznachrichten (SMS) erlaubt. Allein entscheidend ist, dass beide Komponenten auf den bekannten Mobiltelefon-Techniken beruhen, die nach einem bekannten Standard, beispielsweise GSM oder UTSM arbeiten, so dass die bereits vorhandenen Kommunikationsnetze genutzt werden können. Daher kann die erfindungsgemäße extrakorporale Blutbehandlungsvorrichtung mit einem verhältnismäßig geringen technischen Aufwand realisiert werden.

Anstelle einer Kurznachricht kann als Initiierungscode auch eine Sprachnachricht, d.h. ein vom Patienten gesprochenes Wort oder eine Folge von Wörtern (beispielsweise ein Satz) verwandt werden. Auch dies kann vorteilhafterweise mit der bekannten Mobiltelefon-Technologie auf einfache Weise realisiert werden. Wenn der Initierungscode eine Sprachnachricht ist, kann über die Autorisierung durch die Verwendung des Mobiltelefons hinaus eine weitere Autorisierung mit den bekannten Einrichtungen zur Spracherkennung erfolgen.

Im Folgenden wird ein Ausführungsbeispiel der Erfindung unter Bezugnahme auf die anliegende Figur näher erläutert, die in stark vereinfachter Darstellung eine Anordnung aus einer extrakorporalen Blutbehandlungsvorrichtung, eines Kommunikationsnetzes und einer externen Kommunikationseinheit zeigt.

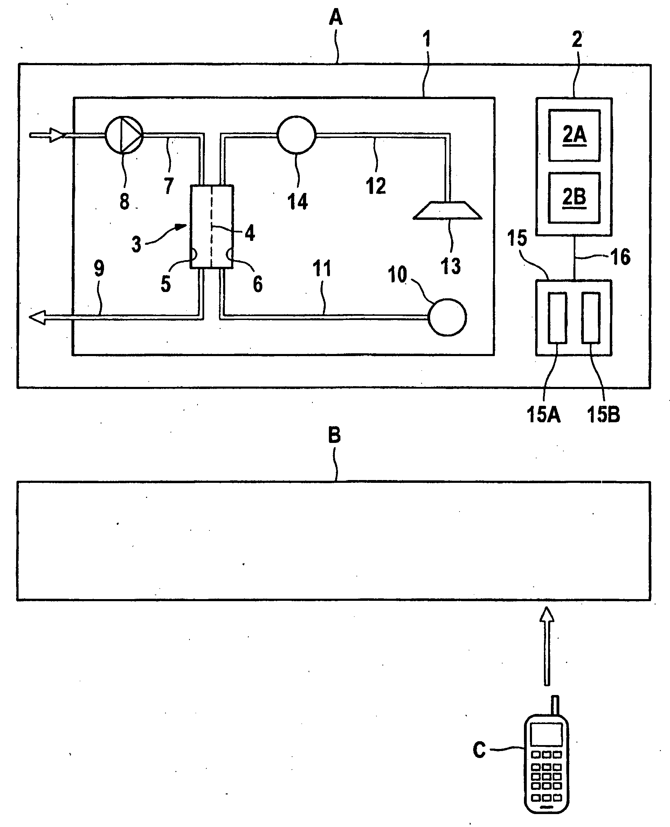

Die Figur zeigt nur die wesentlichen Komponenten der extrakorporalen Blutbehandlungsvorrichtung, da extrakorporale Blutbehandlungsvorrichtungen zur Hämodialyse, Hämofiltration und Hämodiafiltration als solche dem Fachmann bekannt sind.

Die extrakorporale Blutbehandlungsvorrichtung A, beispielsweise eine Hämodialysemaschine, verfügt über eine Behandlungseinheit 1 und eine Einrichtung zur Steuerung der Behandlungseinheit 2. Unter der Behandlungseinheit 1 sind sämtliche Komponenten der Dialysemaschine zu verstehen, die zur Durchführung der eigentlichen Dialyse dienen, während die Steuerungseinrichtung 2 sämtliche Komponenten umfasst, mit denen die für die Durchführung der Dialyse erforderlichen Komponenten gesteuert werden.

Zunächst werden die wesentlichen Komponenten der Behandlungseinheit kurz beschrieben. Die Behandlungseinheit 1 weist einen Dialysator 3 auf, der durch eine semipermeable Membran 4 in eine Blutkammer 5 und eine Dialysierflüssigkeitskammer 6 unterteilt ist. Von einem Patienten führt eine arterielle Blutleitung 7, in die eine Blutpumpe 8 geschaltet ist, zu einem Einlass der Blutkammer 5, während von einem Auslass der Blutkammer eine venöse Blutleitung 9 zu dem Patienten führt.

In einer Dialysierflüssigkeitsquelle 10 wird frische Dialysierflüssigkeit bereitgestellt. Von der Dialysierflüssigkeitsquelle 10 führt eine Dialysierflüssigkeitszuführleitung 11 zu einem Einlass der Dialysierflüssigkeitskammer 6 des Dialysators 2, während eine Dialysierflüssigkeitsabführleitung 12 von einem Auslass der Dialysierflüssigkeitskammer zu einem Abfluss 13 führt. In die Dialysierflüssigkeitsabführleitung 12 ist eine Dialysierflüssigkeitspumpe 14 geschaltet.

Bei der Steuerungseinrichtung 2 kann es sich um einen Computer mit entsprechenden Hardware-Komponenten handeln, auf dem ein Steuerungsprogramm läuft. Die Steuerungseinrichtung übernimmt sowohl die Steuerung der Behandlungseinheit für die Durchführung der Dialyse als auch die Steuerung der Behandlungseinheit für die Vorbereitung der Dialyse. Die Steuerungseinrichtung 2A für die Durchführung der Dialyse und die Steuerungseinrichtung 2B für die Vorbereitung der Dialyse sind schematisch dargestellt.

Darüber hinaus verfügt die Dialysemaschine über eine interne Kommunikationseinheit 15 zur Kommunikation mit einer externen Kommunikationseinheit. Die interne Kommunikationseinheit 15 tauscht mit der Steuerungseinrichtung 2 Daten über eine Datenleitung 16 aus.

Für die Vorbereitung der Dialyse steuert die Steuerungseinrichtung 2B verschiedene Komponenten der Blutbehandlungseinheit 1 an, um die für die Dialyse vor dem Anschluss des Patienten an die Blutleitungen 7, 9 erforderlichen Maßnahmen zu treffen. Diese Maßnahmen hängen von dem Automatisierungsgrad der Dialysemaschine ab, die nur abstrakt beschrieben wird. Vorzugsweise sollte die Dialysemaschine sämtliche Vorbereitungsprozeduren automatisch ausführen, wenn die Steuerungseinrichtung 2B gestartet wird.

Nach der Vorbereitung der Behandlungseinheit 1 für die Dialyse und den Anschluss des Patienten an die Blutleitungen 7, 9 wird die Steuerungseinrichtung 2A zur Durchführung der Dialyse gestartet.

Das Blutbehandlungssystem umfasst neben der Blutbehandlungsvorrichtung A ein Kommunikationsnetz B sowie eine externe Kommunikationseinheit C, die dem Patienten zur Verfügung steht. Grundsätzlich können dem Patienten auch mehrere Kommunikationseinheiten zur Verfügung stehen.

Bei der externen Kommunikationseinheit C sowie dem Kommunikationsnetz B handelt es sich vorzugsweise um ein konventionelles Mobiltelefon (Handy) sowie ein konventionelles Mobilfunknetz. Mit dem Mobiltelefon C kann der Patient über das Mobilfunknetz B mit der internen Kommunikationseinheit 15 der Blutbehandlungsvorrichtung A kommunizieren. Für den Fall, dass die externe Kommunikationseinheit C ein Mobiltelefon ist, weist die interne Kommunikationseinheit 15 ein konventionelles Modul auf, das in den bekannten Mobiltelefonen eingesetzt wird.

Das erfindungsgemäße Blutbehandlungssystem arbeitet wie folgt. Der Patient sendet mit dem Mobiltelefon C eine Kurznachricht (SMS) über das Kommunikationsnetz B an die interne Kommunikationseinheit 15 der Blutbehandlungsvorrichtung A, um die Blutbehandlungsvorrichtung für die Blutbehandlung vorzubereiten. Daraufhin startet die interne Kommunikationseinheit 15 die Steuerungseinrichtung 2A zur Vorbereitung der Dialyse, so dass die Dialysemaschine beispielsweise mit Spülflüssigkeit gespült und mit Dialysierflüssigkeit befüllt wird sowie verschiedene Testroutinen durchgeführt werden.

Das erfindungsgemäße Behandlungssystem ist insbesondere bei der Heim-Dialyse von Vorteil, wo kein Personal für die Einrichtung der Dialysemaschine zur Verfügung steht. Während der Vorbereitung der Dialysebehandlurig braucht der Patient nicht anwesend zu sein. Da dem Patient die Zeitdauer der Dialysevorbereitung bekannt ist, sendet der Patient die Kurznachricht (SMS) dann, wenn er weiß, dass er nach Ablauf dieser Zeitdauer zu Hause ist.

Die interne Kommunikationseinheit 15 startet die Vorbereitung der Dialyse nur dann, wenn ein bestimmter Initiierungscode via Kurznachricht (SMS) empfangen wird. Diese kann vorher festgelegt werden. Es können auch verschiedene Kurznachrichten festgelegt werden, um unterschiedliche Vorbereitungsroutinen zu starten.

Eine alternative Ausführungsform der Erfindung sieht vor, die Vorbereitung der Dialyse nicht mittels einer Kurznachricht (SMS) zu starten, sondern einer Sprachnachricht, weshalb auch bei der alternativen Ausführungsform vorzugsweise wieder ein konventionelles Mobiltelefon als externe Kommunikationseinheit C zum Einsatz kommen kann. Anstelle einer Kurznachricht (SMS) wird nunmehr eine Sprachnachricht empfangen und ausgewertet.

Um sicher zu stellen, dass die Vorbereitung der Dialysemaschine nur von einem bestimmten Patienten mittels eines Mobil-Telefons gestartet werden kann, enthält der Initiierungscode einen Code zur Identifikation des Patienten. Die interne Kommunikationseinheit 15 weist eine Einrichtung 15A zum Speichern eines Codes zur Identifikation eines bestimmten Patienten und eine Vergleichseinheit 15B zum Vergleichen des in dem Initiierungscode enthaltenden Codes zur Identifikation des Patienten auf, beispielsweise einer bestimmten alphanumerischen Zeichenfolge als Teil der SMS.

Wenn die interne Kommunikationseinheit 15 den vom Patienten mittels der externen Kommunikationseinheit C gesendeten Identifikationscode empfängt, vergleicht die Vergleichseinheit 15B den empfangenen Code mit dem gespeicherten Code, um zu prüfen, ob der empfangene Identifikationscode dem gespeicherten Identifikationscode entspricht. Nur wenn dies der Fall ist, startet die interne Kommunikationseinheit die Steuerungseinrichtung 2B für die Vorbereitung der Blutbehandlung.

## Patentansprüche

1. Extrakorporale Blutbehandlungsvorrichtung mit
einer Behandlungseinheit (1) zur Durchführung der extrakorporalen Blutbehandlung,
einer Einrichtung (2A) zur Steuerung der Behandlungseinheit, die derart ausgebildet ist, dass die extrakorporale Blutbehandlung durchgeführt wird,
einer Einrichtung (2B) zur Steuerung der Behandlungseinheit, die derart ausgebildet ist, dass für die Vorbereitung der extrakorporalen Blutbehandlung automatisierte Prozesse durchgeführt werden, und
einer internen Kommunikationseinheit (15), die zur Kommunikation mit einer externen Kommunikationseinheit über ein Kommunikationsnetz ausgebildet ist,
**dadurch gekennzeichnet, dass** die interne Kommunikationseinheit (15) und die Einrichtung (2B) zur Steuerung der Behandlungseinheit für die Vorbereitung der extrakorporalen Blutbehandlung derart ausgebildet sind, dass nach Empfang eines über das Kommunikationsnetz übertragenen Initiierungscodes von der externen Kommunikationseinheit die Einrichtung zur Steuerung der Vorbereitung der extrakorporalen Blutbehandlung gestartet wird, so dass die Behandlungseinheit (1) für die Durchführung der extrakorporalen Blutbehandlung vorbereitet wird.

2. Extrakorporale Blutbehandlungsvorrichtung nach Anspruch 1, **dadurch gekennzeichnet dass** die interne Kommunikationseinheit (15) eine Speichereinheit (15A) zum Speichern eines Codes zur Identifikation eines Patienten und eine Vergleichseinheit (15B) zum Vergleichen eines in dem Initiierungscode enthaltenen Codes zur Identifikation eines bestimmten Patienten mit dem in der Speichereinheit gespeicherten Identifikationscode aufweist.

3. Extrakorporale Blutbehandlungsvorrichtung nach Anspruch 2, **dadurch gekennzeichnet, dass** die interne Kommunikationseinheit (15) und die Einrichtung (2B) zur Steuerung der Behandlungseinheit für die Vorbereitung der extrakorporalen Blutbehandlung derart ausgebildet sind, dass bei einer Übereinstimmung des in dem Initiierungscode enthaltenen Codes zur Identifikation des Patienten mit dem in der Speichereinheit (15A) gespeicherten Identifikationscode die Einrichtung zur Vorbereitung der extrakorporalen Blutbehandlung gestartet wird.

4. Extrakorporale Blutbehandlungsvorrichtung nach einem der Ansprüche 1 bis 3, **dadurch gekennzeichnet, dass** die interne Kommunikationseinheit (15) als eine Mobiltelefon-Einheit ausgebildet ist, wobei der Initiierungscode eine von einer als Mobiltelefon ausgebildeten externen Kommunikationseinheit über ein Mobilfunk-Netz als Kommunikationsnetz gesendete Kurznachricht im SMS-Format ist.

5. Extrakorporale Blutbehandlungsvorrichtung nach einem der Ansprüche 1 bis 4, **dadurch gekennzeichnet, dass** die interne Kommunikationseinheit (15) als eine Mobiltelefon-Einheit ausgebildet ist, wobei der Initiierungscode eine von einer als Mobiltelefon ausgebildeten externen Kommunikationseinheit über ein Mobilfunk-Netz als Kommunikationsnetz gesendete Sprachnachricht ist.

6. Blutbehandlungssystem mit einer extrakorporalen Blutbehandlungsvorrichtung (A) nach einem der Ansprüche 1 bis 5, einem Kommunikationsnetz (B) und einer externen Kommunikationseinheit (C) zur Kommunikation mit der internen Kommunikationseinheit über das Kommunikationsnetz.

7. Blutbehandlungssystem nach Anspruch 6, **dadurch gekennzeichnet, dass** die externe Kommunikationseinheit (C) ein Mobiltelefon und das Kommunikationsnetz (B) ein Mobilfunk-Netz ist.

8. Verfahren zur Vorbereitung einer Blutbehandlung mit einer extrakorporalen Blutbehandlungsvorrichtung, wobei die extrakorporale Bludbehandlungavorrichtung aufweist,
eine Behandlungseinheit (1) zur Durchführung der extrakorporalen Blutbehandlung,
eine Einrichtung (2A) zur Steuerung der Bchandlungseinheit, die derart ausgebildet ist, dass die extrakorporale Blutbehandlung durchgeführt wird,
eine Einrichtung (2B) zur Steuerung der Behandlungseinheit, die derart ausgebildet ist, dass für die Vorbereitung der extrakorporalen Blutbehandlung automatisierte Prozesse durchgeführt werden, und
eine internen Kommunikationseinheit (15), die zur Kommunikation mit einer externen Kommunikationseinheit über ein Kommunikationsnetz ausgebildet ist,
**dadurch gekennzeichnet, dass** von der externen Kommunikationseinheit über das Kommunikationsnetz ein Initiierungscode gesendet wird, wobei nach Empfang des über das Kommunikationsnetz übertragenen Initiierungscodes die Einrichtung zur Vorbereitung der extrakorporalen Blutbehandlung gestartet wird, so dass die Behandlungseinheit für die Durchführung der extrakorporalen Blutbehandlung vorbereitet wird.

9. Verfahren zur Vorbereitung einer Blutbehandlung mit einer extrakorporalen Blutbehandlungsvorrichtung nach Anspruch 8, **dadurch gekennzeichnet, dass** bei einer Übereinstimmung des in dem Initiierungscode enthaltenen Codes zur Identifikation eines bestimmten Patienten mit einem gespeicherten Identifikationscode die Einrichtung zur Vorbereitung der extrakorporalen Blutbehandlung gestartet wird.

10. Verfahren zur Vorbereitung einer Blutbehandlung mit einer extrakorporalen Blutbehandlungsvorrichtung nach Anspruch 8 oder 9, **dadurch gekennzeichnet, dass** als Initiierungscode eine Kurznachricht im SMS-Format von einer als Mobiltelefon ausgebildeten externen Kommunikationseinheit über ein Mobilfunk-Netz als Kommunikationsnetz gesendet wird.

11. Verfahren zur Vorbereitung einer Blutbehandlung mit einer extrakorporalen Blutbehandlungsvorrichtung nach Anspruch 8 oder 9, **dadurch gekennzeichnet, dass** als Initüerungscode eine Sprachnachricht von einer als Mobiltelefon ausgebildeten externen Kommunikationseinheit über ein Mobilfunk-Netz als Kommunikationsnetz gesendet wird.

## Claims

1. Extracorporeal blood treatment apparatus comprising a treatment unit (1) for carrying out the extracorporeal blood treatment, a device (2A) which is intended for controlling the treatment unit and is designed such that the extracorporeal blood treatment is carried out, a device (2B) which is intended for controlling the treatment unit and is designed such that automatic processes for preparing for the extracorporeal blood treatment are carried out, and an internal communication unit (15) designed to communicate with an external communication unit via a communication network, **characterised in that** the internal communication unit (15) and the device (2B) for controlling the treatment unit for the preparation for the extracorporeal blood treatment are designed such that the device for controlling the preparation for the extracorporeal blood treatment is started once an initiation code transmitted via a communication network has been received from the external communication unit, and therefore the treatment unit (1) is prepared for carrying out the extracorporeal blood treatment.

2. Extracorporeal blood treatment apparatus according to claim 1, **characterised in that** the internal communication unit (15) comprises a memory unit (15A) for storing a code for identifying a patient, and a comparison unit (15B) for comparing a code, contained in the initiation code and intended for identifying a particular patient, with the identification code stored in the memory unit.

3. Extracorporeal blood treatment apparatus according to claim 2, **characterised in** - that the internal communication unit (15) and the device (2B) for controlling the treatment unit for the preparation for the extracorporeal blood treatment are designed such that the device for preparing for the extracorporeal blood treatment is started if the code which is contained in the initiation code and intended for identifying the patient matches the identification code stored in the memory unit (15A).

4. Extracorporeal blood treatment apparatus according to any of claims 1 to 3, **characterised in that** the internal communication unit (15) is in the form of a mobile telephone unit, the initiation code being a short message in SMS format which is sent from an external communication unit, in the form of a mobile telephone, via a mobile network.

5. Extracorporeal blood treatment apparatus according to any of claims 1 to 4, **characterised in that** the internal communication unit (15) is in the form of a mobile telephone unit, the initiation code being a voice message which is sent from an external communication unit, in the form of a mobile telephone, via a mobile network.

6. Blood treatment system comprising an extracorporeal blood treatment apparatus (A) according to any of claims 1 to 5, a communication network (B) and an external communication unit (C) for communicating with the internal communication unit via the communication network.

7. Blood treatment system according to claim 6, **characterised in that** the external communication unit (C) is a mobile telephone and the communication network (B) is a mobile network.

8. Method for preparing for a blood treatment by means of an extracorporeal blood treatment apparatus, the extracorporeal blood treatment apparatus comprising a treatment unit (1) for carrying out the extracorporeal blood treatment, a device (2A) which is intended for controlling the treatment unit and is designed such that the extracorporeal blood treatment is carried out, a device (2B) which is intended for controlling the treatment unit and is designed such that automated processes for preparing for the extracorporeal blood treatment are carried out, and an internal communication unit (15) designed to communicate with an external communication unit via a communication network, **characterised in that** an initiation code is sent from the external communication unit via the communication network, the device for preparing for the extracorporeal blood treatment being started once the initiation code transmitted via the communication network has been received, and therefore the treatment unit is prepared for carrying out the extracorporeal blood treatment.

9. Method for preparing for a blood treatment by means of an extracorporeal blood treatment apparatus according to claim 8, **characterised in that** the device for preparing for the extracorporeal blood treatment is started when the code which is contained in the initiation code and is intended for identifying a particular patient matches a stored identification code.

10. Method for preparing for a blood treatment by means of an extracorporeal blood treatment apparatus according to either claim 8 or claim 9, **characterised in that** a short message in SMS format is sent as the initiation code from an external communication unit, in the form of a mobile telephone, via a mobile network acting as the communication network.

11. Method for preparing for a blood treatment by means of an extracorporeal blood treatment apparatus according to either claim 8 or claim 9, **characterised in that** a voice message is sent as the initiation code from an external communication unit, in the form of a mobile telephone, via a mobile network acting as the communication network.

## Revendications

1. Dispositif de traitement extracorporel du sang avec
une unité de traitement (1) pour la réalisation du traitement extracorporel du sang, un dispositif (2A) pour le contrôle de l'unité de traitement qui est conçue de façon à ce que le traitement extracorporel du sang soit effectuée,
un dispositif (2B) pour le contrôle de l'unité de traitement, qui est conçu de façon à ce que, pour la préparation du traitement extracorporel du sang, des processus automatisés soient effectués, et
une unité de communication interne (15) qui est conçue pour communiquer avec une unité de communication externe par l'intermédiaire d'un réseau de communication,
**caractérisé en ce que** l'unité de communication interne (15) et le dispositif (2B) pour le contrôle de l'unité de traitement pour la préparation du traitement extracorporel du sang sont conçus de façon à ce que, après la réception d'un code d'initialisation transmis par l'unité de communication externe, par l'intermédiaire du réseau de communication, le dispositif pour le contrôle de la préparation du traitement extracorporel du sang est démarré, de façon à ce que l'unité de traitement (1) soit préparée pour la réalisation du traitement extracorporel du sang.

2. Dispositif de traitement extracorporel de sang selon la revendication 1, **caractérisé en ce que** l'unité de communication interne (15) comprend une unité de stockage (15A) pour le stockage d'un code pour l'identification d'un patient et une unité de comparaison (15B) pour comparer un code contenu dans le code d'initialisation d'un patient déterminé avec le code d'identification stocké dans l'unité de stockage.

3. Dispositif de traitement extracorporel du sang selon la revendication 2, **caractérisé en ce que** l'unité de communication interne (15) et le dispositif (2B) sont conçus pour le contrôle de l'unité de traitement pour la préparation du traitement extracorporel du sang de façon à ce que, lors d'une correspondance entre le code contenu dans le code d'initialisation pour l'identification du patient et le code d'identification stocké dans l'unité de stockage (15A), le dispositif de préparation du traitement extracorporel du sang est démarré.

4. Dispositif de traitement extracorporel du sang selon l'une des revendications 1 à 3, **caractérisé en ce que** l'unité de communication interne (15) est conçue comme une unité de téléphone mobile, le code d'initialisation étant un message court en format SMS envoyé par une unité de communication externe conçue comme un téléphone mobile par l'intermédiaire d'un réseau de téléphonie mobile en tant que réseau de communication.

5. Dispositif de traitement extracorporel du sang selon l'une des revendications 1 à 4, **caractérisé en ce que** l'unité de communication interne (15) est conçue comme une unité de téléphone mobile, le code d'initialisation étant un message vocal envoyé par une unité de communication externe conçue comme un téléphone mobile par l'intermédiaire d'une unité de communication externe par l'intermédiaire d'un réseau de téléphonie mobile en tant que réseau de communication.

6. Système de traitement de sang avec un dispositif de traitement extracorporel du sang (A) selon l'une des revendications 1 à 5, un réseau de communication (B) et une unité de communication externe (C) pour une communication avec l'unité de communication interne par l'intermédiaire du réseau de communication.

7. Système de traitement de sang selon la revendication 6, **caractérisé en ce que** l'unité de communication externe (C) est un téléphone mobile et le réseau de communication (B) est un réseau de téléphonie mobile.

8. Procédé de préparation d'un traitement de sang avec un dispositif de traitement extracorporel du sang, le dispositif de traitement extracorporel du sang comprenant :
une unité de traitement (1) pour la réalisation du traitement extracorporel du sang,
un dispositif (2A) pour le contrôle de l'unité de traitement, qui est conçu de façon à ce que le traitement extracorporel du sang soit réalisé,
un dispositif (2B) pour le contrôle de l'unité de traitement, qui est conçu de façon à ce que, pour la préparation du traitement extracorporel du sang, des processus automatisés sont effectués, et
une unité de communication interne (15) qui est conçue pour une communication avec une unité de communication externe par l'intermédiaire d'un réseau de communication,
**caractérisé en ce que** l'unité de communication externe envoie, par l'intermédiaire du réseau de communication, un code d'initialisation, le dispositif de préparation du traitement extracorporel du sang étant démarré après réception du code d'initialisation transmis par l'intermédiaire du réseau de communication, de façon à ce que l'unité de traitement soit préparée pour la réalisation du traitement extracorporel du sang.

9. Procédé de préparation d'un traitement du sang avec un dispositif de traitement extracorporel du sang selon la revendication 8, **caractérisé en ce que**, lors d'une correspondance entre le code contenu dans le code d'initialisation pour l'identification d'un patient déterminé et un code d'identification stocké, le dispositif de préparation du traitement extracorporel du sang est démarré.

10. Procédé de préparation d'un traitement de sang avec un dispositif de traitement extracorporel du sang selon la revendication 8 ou 9, **caractérisé en ce que**, en tant que code d'initialisation, un message court est envoyé en format SMS par une unité de communication externe conçue comme un téléphone mobile par l'intermédiaire d'un réseau de téléphonie mobile en tant que réseau de communication.

11. Procédé de préparation d'un traitement du sang avec un dispositif de traitement extracorporel du sang selon la revendication 8 ou 9, **caractérisé en ce que**, en tant que code d'initialisation, un message vocal est envoyé par une unité de communication externe conçue comme un téléphone mobile par l'intermédiaire d'un réseau de téléphonie mobile en tant que réseau de communication.
